# EUROPEAN PATENT APPLICATION

(11) **EP 4 821 455 A1**
(43) Date of publication of application: **14.10.2026**
(21) Application number: 26168849.3
(22) Date of filing: 30.03.2026
(51) Int. Cl.: A61B 34/20, A61B 17/16

(54) **SURGICAL PIN DRIVER SYSTEM AND METHODS**

(30) Priority: 07.04.2025 US 202563784443 P
(71) Applicant: STRYKER CORPORATION, Portage, MI 49002-9711 (US)
(72) Inventor: MCCARTHY, Connor, Kalamazoo, MI 49009 (US); ORR, Haleigh, Spring Lake, MI 49456 (US)
(74) Representative: Röthinger, Rainer

(57) **Abstract**

The present teachings generally provide for a surgical system including a surgical tool configured to guide and drive a fixation device into a target anatomy of a patient along a trajectory. The surgical tool may be configured to support the fixation device along one or more portions of the length of the fixation device in order to increase accuracy and decrease bending of the fixation device during the procedure. The surgical tool may be configured to be tracked by a navigation system. The surgical instrument may be configured to be received by a surgical power tool, or may include a handle for manual use.

## Description

### CROSS-REFERENCE SECTION

The present application claims priority to and all the benefits of U.S. Provisional Patent Application No. 63/784,443, filed on Apr. 07, 2025, the entire contents of which are expressly incorporated herein by reference.

### BACKGROUND

Navigation systems are frequently utilized to assist medical professionals in carrying out various types of surgical procedures, including neurosurgical and orthopedic procedures. To this end, a surgeon may utilize a navigation system to track, monitor, or otherwise locate one or more tools, surgical instruments, and/or portions of a patient's anatomy within a common reference frame. Typically, tools and/or surgical instruments are tracked together with the anatomy, and their relative movement is depicted on a display.

Conventional navigation systems may employ light signals, sound waves, magnetic fields, radio frequency signals, and the like, in order to track the position and/or orientation of objects, such as a surgical instrument or surgical robot relative to the patient and/or surgical site. Often, trackers are attached or otherwise integrated into the object being tracked. A localizer cooperates with tracking elements (e.g., fiducials, markers, and the like) coupled to the tracker to monitor the tracker, and ultimately to determine a position and/or orientation of the object being tracked.

For certain procedures, patient-specific imaging data may be acquired intraoperatively using one or more types of imaging systems to help assist the surgeon in visualizing, navigating relative to, and/or treating the anatomy. To this end, navigation systems may cooperate with imaging systems and/or other parts of surgical systems (e.g., surgical tools, instruments, surgical robots, and the like) to track objects relative to a target site of the anatomy.

In certain surgical procedures, such as those performed using a surgical instrument and/or a surgical robot to manipulate biological tissue and/or place an implant in the patient, trackers may be secured to the surgical instrument and/or surgical robot to track the pose of the surgical instrument or surgical robot relative to the target site of the anatomy.

During reverse shoulder arthroplasty (RSA) procedures, cases are assessed through pre-operative imaging or through surface digitization as a routine diagnostic step to determine which type of implant is most appropriate and optimize implant positioning to maximize the likelihood of positive patient outcomes. Planning software packages allow this data to be further utilized to visualize the implant-anatomic interfaces and plan the intra-operative surgical approach.

Typically, RSA procedures are performed with manual instrumentation that rely on a temporary pin driven into the glenoid over which all subsequent tools are placed to affix the position and trajectory of the glenoid implant, making the initial positioning of the pin a critical step in the full procedure.

### SUMMARY

The present disclosure provides a surgical pin driver system including a pin driver and an adapter for mounting a tracker to the pin driver for use with a surgical navigation system to track the pose of the surgical instrument and assist with aligning to a target trajectory.

One general aspect includes a surgical system for navigated surgery. The surgical system also includes a surgical power tool with a housing including a motor and a receiver, the motor disposed within the housing, the motor operatively connected with the receiver, the receiver configured to removably receive a tool. The system also includes a tool including: a body with a distal portion with a distal end and a proximal portion with a proximal end, the proximal portion of the body configured to be selectively received by the receiver of the surgical power tool; a lumen defined through the body by an inner wall forming a void and extending between the distal end and the proximal end of the body; and a retention assembly configured to selectively retain a fixation device. The system also includes a navigation adapter. The system also includes a fixation device. Wherein when the tool is inserted into the surgical power tool, the proximal end of the tool is positioned within the receiver of the surgical power tool and the fixation device is retained by the retention assembly is positioned such that the proximal end of the fixation device is supported by the tool within the surgical power tool.

Implementations may include one or more of the following features: The receiver of the surgical power tool is configured to impart rotational movement from the motor to tool, where the tool is configured to apply a torque to the fixation device through a drive portion. The drive portion includes a keyed cross-section and a retention assembly. The retention assembly may include a biasing element. The proximal end of the fixation device may include a geometry substantially matching the keyed cross-section of the drive portion. The drive portion is within the proximal portion of the body. An inner diameter of the drive portion is sized and shaped to support a length of the proximal portion of the fixation device. The proximal portion of the tool includes an outer profile that is sized and shaped to be received within and supported by the surgical power tool, the outer profile of the proximal portion including a first outer diameter configured to interface with a spindle of the receiver of the surgical power tool, and a second outer diameter is greater than the first outer diameter configured to interface with a receiver retainer of the surgical power tool. The system the outer profile of the proximal end of the tool further including a third outer diameter greater than the second outer diameter and configured to receive the navigation adapter, and a fourth outer diameter larger than the third outer diameter and configured as a positional stop for the navigation adapter. The distal portion of the tool includes a first inner diameter and a second inner diameter smaller than the first inner diameter extending to the distal end of the tool, the second inner diameter providing limited radial compliance with the fixation device to support the proximal portion of the fixation device. The distal portion of the tool includes a handle section and a nose tube, the handle section having a handle outer diameter and the nose tube having a tapered diameter reducing in size from the handle outer diameter to the distal end of the tool. The body of the tool may include a first body corresponding to the proximal portion and a second body corresponding to the distal portion. The system may include a navigation adapter configured to support a navigation array, the navigation adapter coupled to the tool and the surgical power tool. The fixation device has a length longer than the tool. The fixation device may be a pin or a guide wire.

One general aspect includes a surgical tool configured to drive a fixation device in a patient. The surgical tool also includes a body with a distal portion and a proximal portion. The tool also includes a lumen defined through the body by an inner wall forming a void between a distal end of the distal portion and a proximal end of the proximal portion of the body, the inner wall configured to support a length of the fixation device within the lumen. The tool also includes a retention assembly configured to selectively retain a fixation device. The tool also includes a sleeve disposed about a portion of the distal end of the body. The tool also includes where the inner wall supports the fixation device at the proximal end and distal end of the tool, the lumen configured to receive a proximal portion and a distal portion of the fixation device such that the inner wall supports the proximal portion and the distal portion of the fixation device.

Implementations may include one or more of the following features. The distal portion of the fixation device may be supported by the tool at least at a distal end of the fixation device. The distal portion of the fixation device is defined as the length between a midpoint of the fixation device to a distal end of the fixation device. The tool is configured to receive a navigation adapter selectively positionable about circumference of the body of the tool. The distal portion of the tool includes an inner diameter extending to the distal end of the tool, the inner diameter providing limited radial compliance with the fixation device to support the proximal portion of the fixation device. The body includes a drive portion, the drive portion configured to rotate the fixation device with the tool.

Other features and advantages of the embodiments of the present disclosure will be readily appreciated, as the same becomes better understood, after reading the subsequent description taken in conjunction with the accompanying drawings.

### BRIEF DESCRIPTION OF THE DRAWINGS

Figure 1 is a perspective view of a surgical system shown comprising a navigation system and an imaging system supporting a patient.
Figure 2 is a perspective view of a surgical instrument driving a pin into a scapula.
Figure 3 illustrates the guide pin driven by the surgical instrument into the scapula.
Figure 4 is a perspective view of the surgical instrument consistent with the teachings herewith.
Figure 5 is sectional view of the surgical instrument and one example of a fixation device.
Figure 6 is an exploded perspective view of the fixation device, the surgical tool, and the rotation adapter of the surgical instrument.
Figure 7 provides for distal end view of the surgical instrument with the retention assembly.
Figure 8 illustrates a side view the fixation device within the retention assembly.
Figure 9 illustrates a perspective view of the retention assembly and the fixation device.
Figures 10 and 11 are perspective views of the instrument tracker mount assembly.
Figure 12 illustrates the surgical instrument and a hand-piece.
Figure 13 is a perspective view of the surgical instrument inserted into the hand-piece.
Figure 14 is a cross sectional view of the surgical instrument inserted into the power tool shown in Figure 13.

It will be appreciated that one or more of the embodiments depicted throughout the drawings may have certain components, structural features, and/or assemblies removed, depicted schematically, and/or shown in phantom for illustrative purposes.

### DETAILED DESCRIPTION

The present teachings generally provide for a surgical system including a surgical tool configured to guide and drive a fixation device into a target anatomy of a patient along a trajectory. The surgical tool may be configured to support the fixation device along one or more portions of the length of the fixation device in order to increase accuracy and decrease bending of the fixation device during the procedure. The surgical tool may be configured to be tracked by a navigation system. The surgical tool may be configured to be received by a surgical power tool, or may include a handle for manual use.

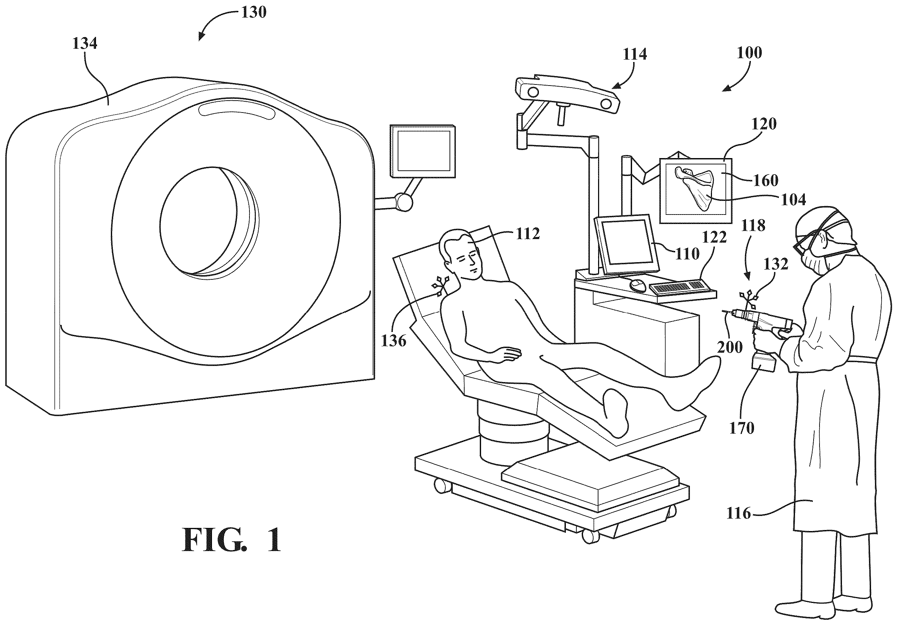

FIG. 1 is a perspective view of the surgical system 100 may be configured to assist a user 116 in visualizing patient shoulder anatomy and/or planning a prospective surgery on a patient 112. The surgical system 100 may include a surgical instrument 118, a display 120, an imaging system 130, a user input device 122, and a navigation system 114. The surgical system 100 may include a controller 110 that is configured to communicate with the imaging system 130, the display 120, the user input device 122, and/or the navigation system 114. For example, there may be a wired or wireless connection between the controller 110, the imaging system 130, the navigation system 114, the user input device 122, and the display 120.

The controller 110 may function to control one or more of the various elements 130, 114, 122, 120 of the surgical system 100. In some examples, the surgical system may include a plurality of controllers, each connected to and communicating with each other the surgical instrument 118, the display 120, the imaging system 130, the user input device 122, and the navigation system 114. The controller 110 may be part of a personal computer, laptop computer, tablet computer, other suitable computing device, or a plurality of suitable computing devices. In some implementations, the controller 110 may be integrated with the display 120 or the imaging system 130, or it may be implemented by multiple elements of the surgical planning system 100. The controller 110 may be configured to receive, send, or process data between the display 120 and the imaging system 130, or other computing devices. Further, the controller 110 may be configured to receive input from the user input device 122 to run software, send data, receive data, or manipulate the data shown on the display 120.

The controller 110 may utilize a segmentation algorithm, such as a deep learning model, to generate the 3D models of patient shoulder anatomy from the image data. The processor may be configured to use the software to generate a two-dimensional and/or three-dimensional model including at least a portion of patient shoulder anatomy from the image data. The imaging display may be configured to display the 3D models generated by the processor. The 3D model may further include indicators corresponding to anatomical locations, or virtual objects representing anatomical landmarks, or other modifications not specifically described herein.

The imaging system 130 may include an imaging device, such as a CT machine 134, an MRI machine, an X-ray machine, or any other type of intra-operative and/or pre-operative imaging device, along with a display and computer. While the illustrated imaging system 130 is realized as an x-ray CT imaging device as noted above, in other embodiments, the imaging system 130 may comprise one or more of an x-ray fluoroscopic imaging device, a magnetic resonance (MR) imaging device, a positron emission tomography (PET) imaging device, a single-photon emission computed tomography (SPECT), or an ultrasound imaging device. In some other examples, the imaging system 130 may be a C-arm x-ray fluoroscopy device. Other configurations are contemplated. For example, when the imaging device is a CT machine 134, the imaging system 130 may generate CT image data. In some implementations, the imaging device may be further configured to obtain 3D models of the anatomy being imaged, for example the patient shoulder anatomy. The imaging system 130 may be configured to generate one or more types of imaging data or combine types of imaging data. The imaging system 130 may transmit the image data obtained by the imaging device to the controller 110 to be processed, displayed, or otherwise manipulated by the controller 110.

An example imaging system 130 that may be used in various embodiments is the AIRO^{®} intra-operative CT system manufactured by Mobius Imaging, LLC. Examples of x-ray CT imaging devices that may be used according to various embodiments of the present disclosure are described in U.S. Patent No. 10,151,810, entitled *"Pivoting Multi-directional X-ray Imaging System with a Pair of Diametrically Opposite Vertical Support Columns Tandemly Movable Along a Stationary Base Support;"* U.S. Patent No. 9,962,132, entitled *"Multi-directional X-ray Imaging System with Single Support Column;"* U.S. Patent No. 9,801,592, entitled *"Caster System for Mobile Apparatus;"* U.S. Patent No. 9,111,379, entitled *"Method and System for X-ray CT Imaging;"* U.S. Patent No. 8,118,488, entitled *"Mobile Medical Imaging System and Methods;"* and U.S. Patent Application Publication No. 2014/0275953, entitled *"Mobile X-ray Imaging System,"* the disclosures of each of which are hereby incorporated by reference in their entirety.

In some examples, the controller 110 may be configured to cause the display 120 to show various graphical user interfaces (GUI) 160. Patient image data (e.g., pre-operative patient images or intraoperative patient images) and/or renderings of 3D models of the patient shoulder anatomy may be included within the GUI 160. The display may be configured for the user 116 to interact with elements included in the GUIs 160 (such as renderings of 3D models) via touch input to the display 120, the user input device 122, or via other suitable means. Further, the controller 110 may be configured to cause the display 120 to show representations of anatomical landmarks of the patient shoulder anatomy, measurement values corresponding to the patient shoulder anatomy, and/or representations of reconstructions of the patient shoulder anatomy. In some implementations, the GUI 160 may also be utilized as a display only and allow no interaction from the user 116. The controller 110 may be configured to cause the display 120 to show a surgical plan for a medical procedure or provide options and outcomes for various surgical planning purposes or show other features of the patient shoulder anatomy. Other features of the patient shoulder anatomy exist, and those not specifically discussed herein may also be shown on the display 120. The GUI 160 may include one or more configurations, each of which may include additional views of the image data, the surgical instrument, an implant, surgical planning, steps of a surgical procedure, the like, or a combination thereof. The GUI 160 may be configured to allow the user 116 to manipulate the information shown on the display 120.

In some implementations, the user input device 122 is a keyboard and mouse and allows the user 116 to manipulate objects shown on the display 120 by clicking, dragging, scrolling, typing, or any combination of these actions to provide input to the GUI 160. Additionally, or alternatively, the display 120 may be a touch screen which allows the user 116 to manipulate the renderings of 3D models shown on the display 120 via touch input/gestures. In other examples, the user input device 122 may be a button, a switch, foot pedal, or the like. The display 120 may be a monitor, the screen of a laptop computer, or an AR display. For example, the display 120 may be a headset configured to be worn by the user 116. In some implementations, the display 120 may be configured as an extended reality device configured to execute any of the graphical functions described herein. The extended reality device may be implemented by a hand-held device (e.g., tablet or smart phone) or a head-mounted device. The extended reality device may be configured to superimpose, overlay, or combine any of the described computer-generated graphics with real-world views to implement an extended reality, augmented reality, and/or mixed reality experience for the user 116. The real-world views may be acquired directly by the eyes of the user 116 or may be a real-world video stream captured by one or more cameras of the extended reality device. When a head mounted device is utilized, the head-mounted device may include a transparent lens or one or more display screens positioned directly in front of the eyes of the user 116 to display the computer-generated graphics relative to the real-world views.

In some implementations, the navigation system 114 includes a localizer 115. The localizer 115 may be an optical localizer and includes a camera unit with an outer casing that houses one or more optical sensors configured to sense movement of the various trackers. To this end, any one or more of the trackers may include active markers (not shown in detail). The active markers may include light emitting diodes (LEDs). Alternatively, the trackers may have passive markers, such as reflectors which reflect light emitted from the camera unit or another predetermined light source. In other implementations, the localizer 115 may be electromagnetically (EM) based. For example, the navigation system may include an EM transceiver coupled to the navigation controller. The localizer 115 may also be radio frequency (RF) based. In such a case, the localizer 115 may include an RF transceiver coupled to the navigation controller and/or to the computing device. Here, the trackers may include RF emitters or transponders, which may be passive or may be actively energized. The RF transceiver transmits an RF tracking signal, and the RF emitters respond with RF signals such that tracked states are communicated to (or interpreted by) the navigation controller.

The surgical system 100 employs the navigation system 114 to, among other things, track movement of various objects, such as the surgical instruments 118 and parts of the patient's 112 anatomy (e.g., tissue at the surgical site ST). In some examples, the navigation system 114 may track portions of the imaging system 130. To this end, the navigation system 114 comprises a navigation controller coupled to a localizer 115 that is configured to sense the position and/or orientation of trackers 132, 136 within a localizer coordinate system. As is described in greater detail below, the trackers 132, 136 (also referred to herein as "navigable trackers") are fixed, secured, or otherwise attached to specific objects, and are configured to be monitored by the localizer 115.

In Figure 1, the instrument tracker 132 is depicted generically and are mounted to the surgical instrument 118. Patient tracker 136 may be removably coupled to mount assemblies to define tracker assemblies which facilitate selective movement of the trackers 136 relative to their mount assemblies. Here, the patient is firmly fixed to different portions of the patient's 112 anatomy via anchors which are configured to releasably engage tissue (e.g., bone). It will be appreciated that patient trackers 136 may be firmly affixed to different types of tracked objects (e.g., discrete bones, tools, pointers, and the like) in a number of different ways.

The surgical system 100 includes one or more surgical instruments 118. Figure 1 illustrates surgical instrument 118 as a surgical handpiece 170, shown here as a powered handpiece, with a surgical tool 200 (also known as an end effector), which will be described further below. Although the surgical instrument 118 is shown as a handpiece 170 with surgical tool 200, it is contemplated that the surgical instrument may be a robot end effector, a non-powered tool, and/or other variations of a surgical instrument that the surgical tool 200 may be removably coupled with.

For illustrative purposes, surgical instrument 118 is generally depicted and configured for hand-held use is shown in Figure 1. However, as will be appreciated from the subsequent description below, aspects of the surgical system 100 may be used with any suitable type of surgical instrument 118 without departing from the scope of the present disclosure. Furthermore, aspects of the surgical system 100 may also be employed that may be controlled, guided, positioned, or otherwise manipulated with one or more types of surgical robots. Certain types of surgical robots are disclosed in U.S. Patent No. 9,119,655, entitled *"Surgical Robotic arm Capable of Controlling a Surgical Instrument in Multiple Modes;"* U.S. Patent No. 10,456,207, entitled *"Systems and Tools for use with Surgical Robotic Manipulators;"* U.S. Patent Application Publication No. 2019/0231447, entitled *"End Effectors And Methods For Driving Tools Guided By Surgical Robotic Systems;"* U.S. Patent Application Publication No. 2016/0302871, entitled *"Integrated Medical Imaging and Surgical Robotic System;"* U.S. Patent Application Publication No. 2020/0078097, entitled *"Methods and Systems for Robot-Assisted Surgery;"* and U.S. Patent Application Publication No. 2022/0273396, entitled *"Robotic Hand-held Surgical Instrument Systems and Methods,"* the disclosures of each of which are hereby incorporated by reference in their entirety.

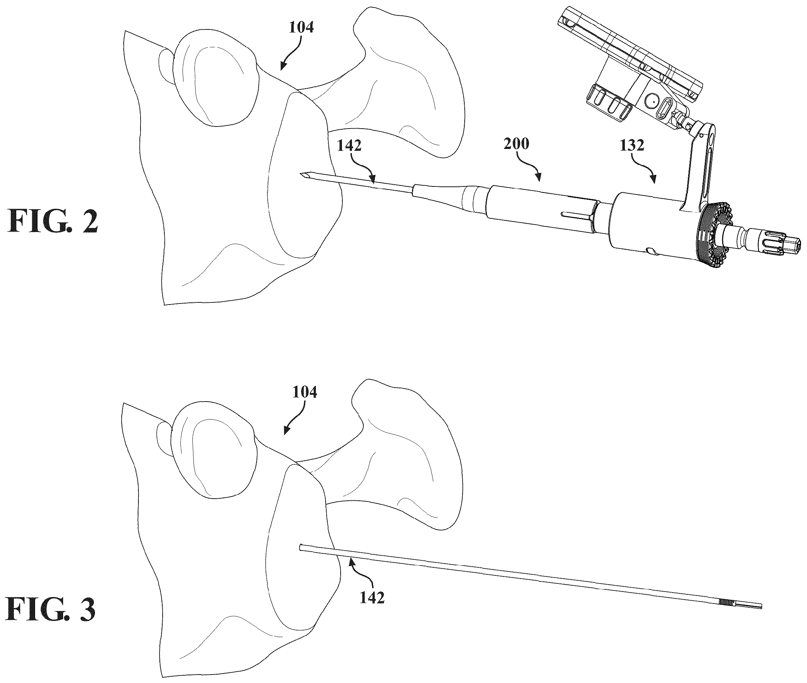

Referring to Figures 2 and 3, the surgical tool 200 is configured for performing a portion of a reverse shoulder arthroplasty surgical procedure on a scapula 104. In some examples, the surgical tool 200 may be used during a shoulder surgery, or any other surgical procedure to attach a fixation device 142 to a patient bone. In this example, fixation device 142 is a guide pin. The guide pin may be configured to guide one or more surgical instruments throughout the procedure, such as a surgical reamer. In some examples, the surgical tool 200 is actively tracked with instrument tracker 132 to assist with placement of the fixation device 142 along a pre-planned trajectory.

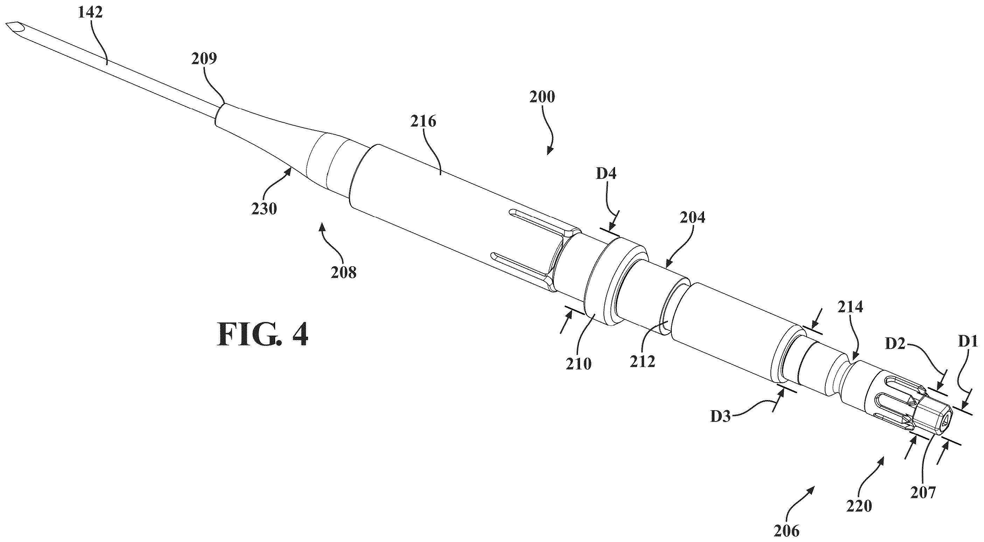

Referring to Figures 4-6, an exemplary configuration of the surgical tool 200 is illustrated. The surgical tool 200 may be configured to be removably connected with the handpiece 170 to facilitate a surgical procedure. The surgical tool 200 may be configured to support a fixation device 142, shown here as a guide pin, and drive the fixation device into a patient at a target area, such as shown in Figures 2 and 3.

Turning to Figure 4, the surgical tool 200 comprises a body 204 having a profile with a proximal portion 206 with a proximal end 207, and a distal portion 208 with a distal end 209. The body 204 may define the profile of the surgical tool 200 and include several features to assist a user during a surgical procedure. The body 204 extends between the proximal end 207 and the distal end 209. In some examples, the surgical tool 200 may be a unitary body. In other examples, the surgical tool 200 may comprise multiple components.

The proximal portion 206 of the body 204 may include several retention features 210, 212, 214 to interface with instrument tracker 132 and handpiece 170. The proximal portion 206 houses the retention assembly 220 configured to receive and retain the fixation device 142, which will be described further below. The proximal portion 206 may include drive features for rotating the surgical tool 200 when connected with the handpiece 170. In some examples, the proximal portion 206 may include a first diameter D1 of the body 204, a second diameter D2 of the body 204 larger than the first diameter D1, and a third diameter D3 of the body 204 which is larger than the second diameter D2. A fourth diameter D4 of the body 204 may be sized to be larger than the third diameter D3 of the body 204. In some examples, the fourth diameter D4 may be configured as stop 210, described further below. It is contemplated that the size, shape, and location of each section of the body 204 may be located at any portion along the body 204 to facilitate attachment and connection with instrument tracker 132, handpiece 170, and/or other surgical components.

The body 204 of the surgical tool 200 may further comprise a retainer 214. The retainer 214 may comprise an annular recess in the proximal portion 206. For example, as illustrated in Figure 4-6, the retainer 214 is realized with a V-shaped recess defined in the proximal portion 206 of the surgical tool 200. As will be described further below, the retainer 214 may be configured to cooperate with the coupling mechanism 180 of the receiver 178 on the handpiece 170 to removably secure the surgical tool 200. While the retainer 214 is illustrated as a recess having a V-shaped profile throughout the drawings, it is contemplated that the recess may be defined in alternate shapes that cooperate with the coupling mechanism 180 of the receiver 178 of the handpiece 170. For example, the retainer 214 may comprise a recess defined in the body 204 having a square-shaped profile, with the opposing surfaces of the recess arranged to be generally parallel to one another.

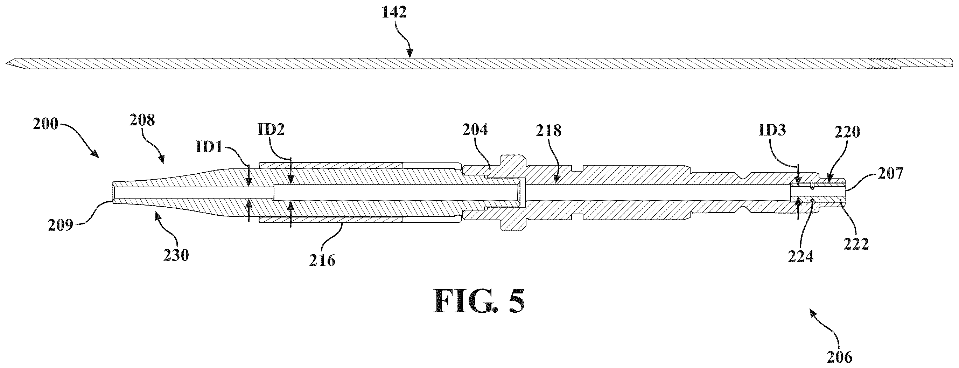

The surgical tool 200 may include a stop 210 arranged between the proximal portion 206 and the distal portion 208. Referring to Figure 5, the stop 210 comprises an annular body disposed on the body 204 of the surgical tool 200. The stop 210 may be formed as part of the body 204 and configured as a protrusion that encircle and extends outwardly from an outer surface of the body 204. In some versions, the stop 210 may be configured as a separate component that is attached to the body 204 of the surgical tool. For example, the stop 210 may comprise a bearing or washer that is coupled to the body 204. The stop 210 may be press-fitted onto the body 204. Attentively, the stop 210 may be coupled to the body 204 via a weld, epoxy, or similar adhesive. It will be appreciated that the stop 210 could be defined by various components and/or structural features. Other configurations are contemplated.

The stop 210 is positioned a defined location between the proximal portion 206 and the distal portion 208 of the surgical tool 200 and is configured to abut a rotational adapter 184 of the instrument tracker 132. For example, the stop 210 may be positioned on the body 204 of the surgical tool 200 at a known distance from the retainer 214. The distance that the stop 210 is spaced from the retainer 214 may be based on the length of the adapter 184. The known distance between the stop 210 and the retainer 214 may be selected to compress a biasing element 196 a known distance when the surgical tool 200 is coupled to the handpiece 170, such as described in U.S. Patent Publication US2024/0225747, titled "Rotational Tracker Adapter With Biasing Mechanism," which is hereby incorporated by reference herein.

The stop 210 may also be configured to position the adapter 184 of the instrument tracker 132 at a known distance from the distal portion 208 of the surgical tool 200. Here, it will be appreciated that positioning the adapter 184 at a known distance from the distal portion 208 of the surgical tool 200 may allow the navigation system 114 to precisely and accurately determine the position and/or orientation of the distal portion 208 of the surgical tool 200 relative to the patient anatomy 104 and/or target object.

The surgical tool 200 may also comprise a recess 212, as described above, that is configured to receive a portion of the adapter 184 of the instrument tracker 132 when attached to the surgical tool 200. The recess 212 on the proximal portion 206 of the surgical tool 200 may be configured to selectively retain the adapter 184 to the surgical tool 200. In some examples, the size and shape of the recess 212 may be defined in the outer surface of the proximal portion 206 of the surgical tool 200 to allow for lateral movement of a portion of adapter 184 with respect to the recess 212 to allow the adapter 184 to compress and cause the adapter 184 to abut the stop 210.

While the illustrated embodiments depict the recess 212 completely encircling the body 204 of the surgical tool 200, it is contemplated that the recess 212 may be configured as a groove or differently-shaped formation that only partially encircles the body 204.

With continued reference to Figures 4-6, the distal portion 208 of the body 204 may include a handle 216 rotatable about the body 204.
The handle is configured to freely rotate about body 204. The handle 216 may function to permit a user to grip and guide the surgical tool 200, as the surgical tool 200 is rotated by handpiece 170. In some examples, the handle 216 may be made of the same material as the surgical tool 200 (e.g., stainless steel). In other examples, the handle 216 may be made of a different material, such as a plastic (e.g., PEEK). It is contemplated that the handle 216 may have any size and/or shaped to facilitate a gripping portion of the surgical tool 200 during operation.

The body 204 may further include a nose cone 230. The nose cone 230 tapers towards the distal end 209, reducing in outer diameter until reaching the distal end 209. The nose cone 230 may function to support and guide the fixation device 142 as the surgical tool 200 drives the fixation device 142 into the patient anatomy 104. More specifically, the distal end 209 of the body 204 may support a distal portion 148 of the fixation device 142 and a proximal end 207 of the body 204 may support a proximal portion 144 of the fixation device 142.

Turning to Figure 5, a cross section of surgical tool 200 is shown, showing the interior profile of body 204. The body 204 includes an inner wall 218 forming a void that defines a lumen which is sized and shaped to receive and support fixation device 142. The inner wall 218 extends from proximal end 207 to distal end 209. Nose cone 230 includes a first inner diameter ID1 which is slightly larger than the diameter of the fixation device 142 to allow minimal radial compliance. Additionally, the first inner diameter ID1 extends a length of the lumen defined by inner wall 218, the inner wall 218 is configured to support a portion of a distal area of the fixation device 142, which may prevent bending and deformation of the fixation device 142 while inserting into the patient anatomy. The inner wall 218 further defines a second inner diameter ID2 which is larger than the first inner diameter ID1. In some examples, the second inner diameter ID2 defined by the inner wall 218 may be configured to allow the fixation device 142 freely pass through the lumen towards the third inner diameter ID3.

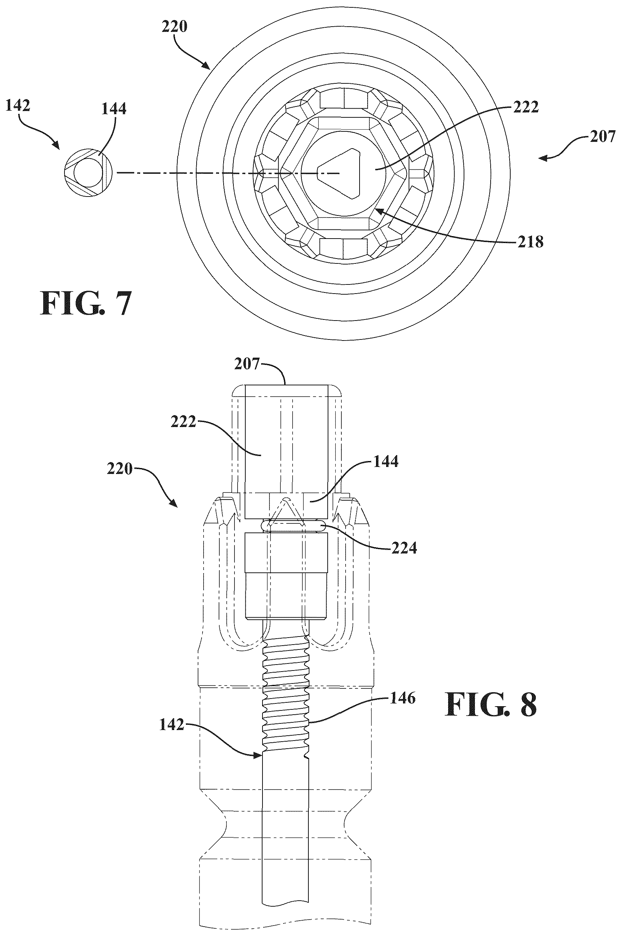

The proximal portion 206 of the body 204 includes a retention assembly 220 configured to removably receive and retain fixation device 142 when the fixation device 142 is loaded into the surgical tool 200. The retention assembly 220 (best seen in Figures 7-9), may function to retain the fixation device 142 axially within the surgical tool. As shown in Figure 5, the retention assembly 220 may be pressed into the lumen defined by inner wall 218. In this example, the outer diameter of the retention assembly 220 may substantially similar and/or slightly larger than the second inner diameter ID2, such that the retention assembly 220 may be press fit into the lumen defined by inner wall 218. Other methods of connecting and securing the retention assembly 220 are contemplated.

The retention assembly 220 may have a third inner diameter ID3 configured to receive the proximal end of the fixation device 142. In some examples, the third inner diameter ID3 may be substantially similar to the first inner diameter ID1 of the distal portion 208. In another example, the third inner diameter ID3 may be smaller than the first inner diameter ID1 of the distal portion 208. The third inner diameter ID3 is sized and shaped to receive the proximal portion of the fixation device 142. In some examples, the retention assembly 220 is sized and shaped to receive and support a proximal portion 144 of the fixation device 142. The retention assembly 220 is configured to provide support to the proximal portion 144 of the fixation device 142 as torque is applied from the surgical tool 200 to the fixation device 142. The inner wall 218 of the surgical tool 200 supports the outside diameter of the fixation device 142 and reduces the chances of bending the fixation device 142 as the fixation device 142 is driven into the target anatomy, significantly improving the accuracy of guide wire placement.

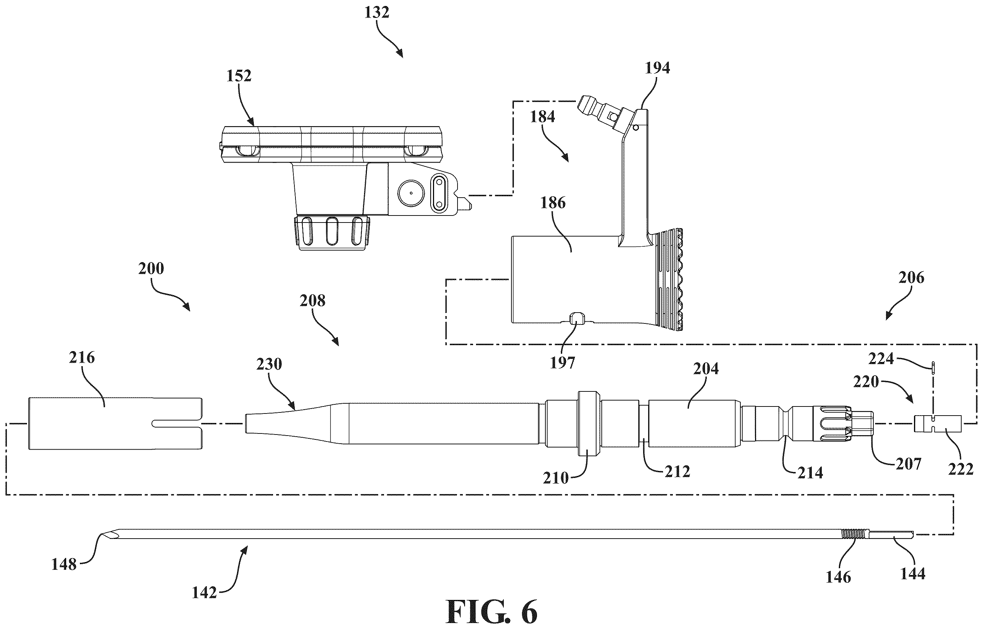

Figure 6 illustrates an exploded view of the surgical tool 200, including instrument tracker 132 and fixation device 142. In this example, a proximal portion 144 of the fixation device 142 may be loaded into the distal end 209 of the body 204 of the surgical tool 200 until the proximal portion 144 of the fixation device 142 is received by the retention assembly 220 at the proximal portion 206 of the surgical tool 200. Further, a distal end of the instrument tracker 132 is slide over the proximal portion 206 of the surgical tool 200 until the instrument tracker 132 is stopped by stop 210, and coupler 197 is received in recess 212 to retain the tracker at a determined axial position.

Figures 7-9 illustrate the proximal portion 206 of the body 204 including the retention assembly 220 when the fixation device 142 is disposed through the lumen defined by inner wall 218 and retained within the surgical tool 200. Figure 7 illustrates an end view of the proximal portion 144 of the fixation device 142 and the retention assembly 220 in the body 204. In this example, the fixation device 142 includes a triangular profile at the proximal portion 144. Similarly, the retention base 222 of the retention assembly 220 includes a throughhole with a triangular cross section configured to receive the triangular profile of the proximal portion 144 of the fixation device 142. Although a triangular example is shown, any suitable shape forming a keyed interface to impart rotational force from the surgical tool 200 onto the fixation device 142 is contemplated.

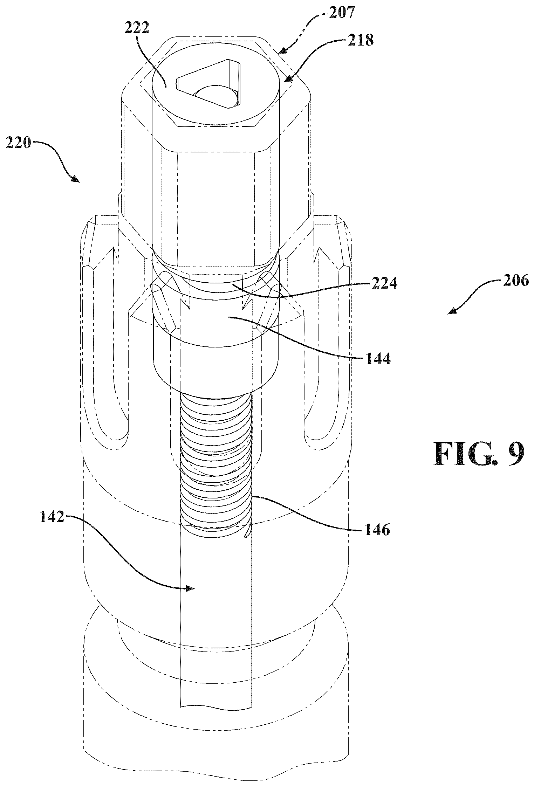

Turning to Figures 8 and 9, the proximal portion 206 of the surgical tool 200 and the proximal portion 144 of the fixation device 142 are shown. In this example, the fixation device 142 is inserted into the lumen defined by inner wall 218 and received by retention assembly 220. The retention assembly 220 axially secures the fixation device 142 to the retention base 222 with clip 224. The clip 224 is configured to removably secure the fixation device 142 to the surgical tool 200 such that a user may drive the fixation device 142 in the target anatomy, and remove the surgical tool 200 by applying a force opposite the target anatomy which will allow the surgical tool 200 to release the fixation device 142. In this example, fixation device 142 includes threads 146 distal to the triangular proximal portion 144.

### TRACKER AND TRACKER MOUNT

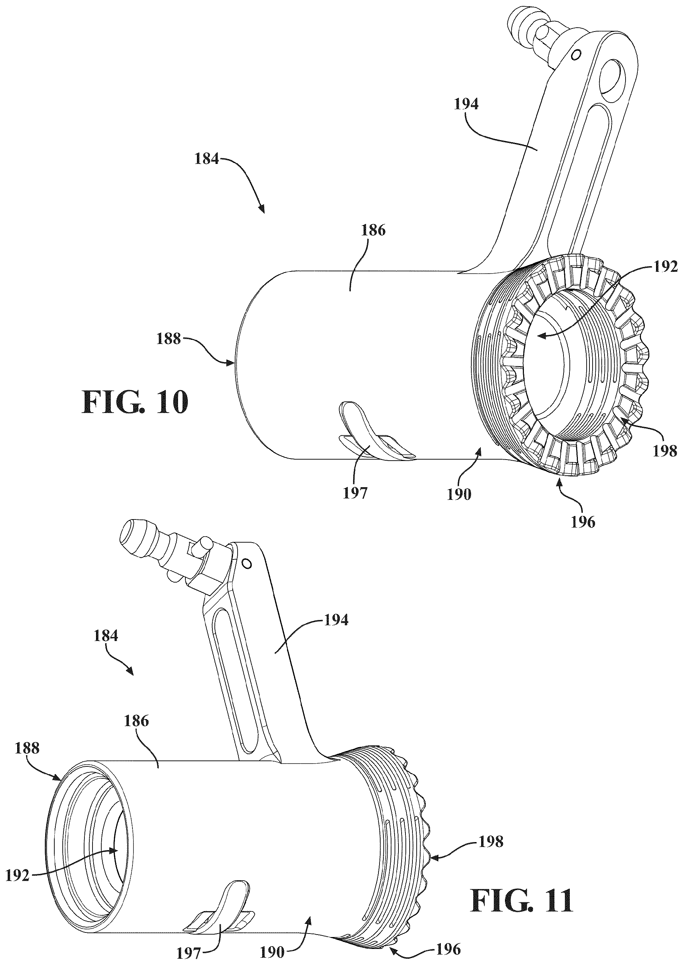

As noted above, during a surgical procedure, an instrument tracker 132 including a marker 152 (also referred to herein as a tracking element) may be coupled to the surgical tool 200 to allow the navigation system 114 to determine the position and/or orientation of the surgical tool 200. Referring to Figure 10 and 11, an exemplary configuration of an adapter 184 for coupling an instrument tracker 132 to a surgical tool 200 is illustrated. The adapter 184 generally includes a base 186 having a distal end 188 and an opposing proximal end 190. The base 186 may also define an aperture 192 that extends through the adapter 184 and is configured to receive a portion of the surgical tool 200. For example, the surgical tool 200 comprises a body 204 extending between a distal portion 208 for manipulating tissue and proximal portion 206 defining a retainer 214 for removably attaching the surgical tool 200 to the surgical handpiece 170. The surgical tool 200 also includes a stop 210 arranged between the proximal portion 206 and the distal portion 208 of the body 204. The aperture in the base 186 of the adapter 184 is defined to receive the body 204 of the proximal portion 206 of the surgical tool 200. The adapter 184 may also include a mount 194 coupled to the base for releasably securing the tracker to the adapter. A biasing element 196 disposed on the base 186 of the adapter 184 includes an abutment interface 198 for engaging the surgical handpiece 170 and is configured to urge the base 186 away from the surgical handpiece 170. The biasing element 196 is compressed in response to attachment of the retainer 214 to the surgical handpiece 170 with abutment occurring between the base 186 and the stop 210 of the surgical tool 200 to limit movement of the adapter 184 relative to the surgical tool 200.

The adapter 184 may also comprise the mount 194 extending from the base 186. The mount 194 may comprise a coupler, such as a quick connect adapter, configured to removably couple the instrument tracker 132 to the adapter 184. In some versions, the coupler may comprise a threaded, a quarter turn, a snap fit, or similar connector for removably coupling the instrument tracker 132 to the adapter 184. While not described in detail herein, in some embodiments, the mount 194 or another portion of the adapter 184A may comprise or otherwise be realized as a coupler similar to as is described in U.S. Patent No. 7,725,162, entitled *"Surgery System,"* the disclosure of which is incorporated by reference in its entirety. Other configurations are contemplated.

### CONNECTING TOOL AND HANDPIECE

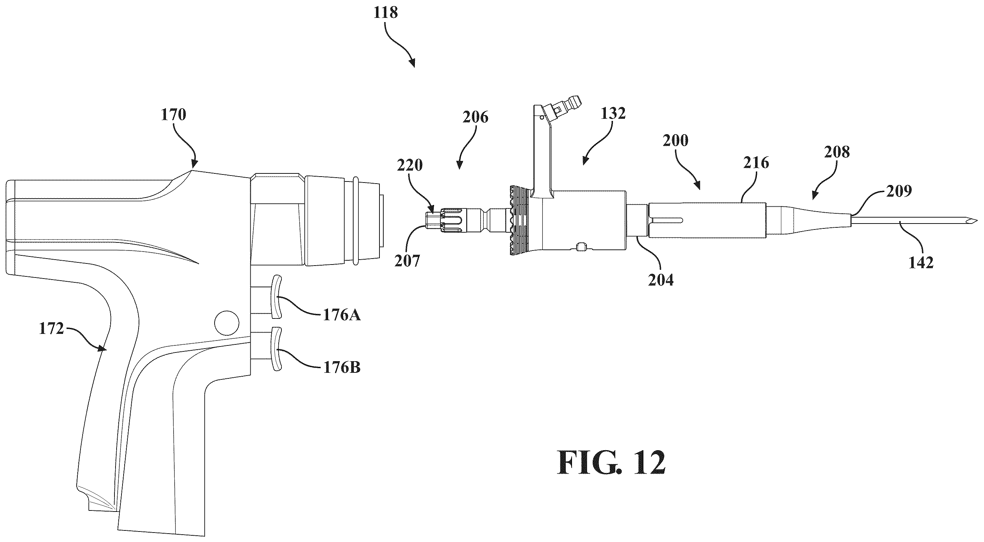

Turning to Figures 12-14, the surgical tool 200 is configured to be attached and received by the surgical handpiece 170. Figure 12 illustrates a sideview of the surgical tool 200 ready to be inserted into the handpiece 170.

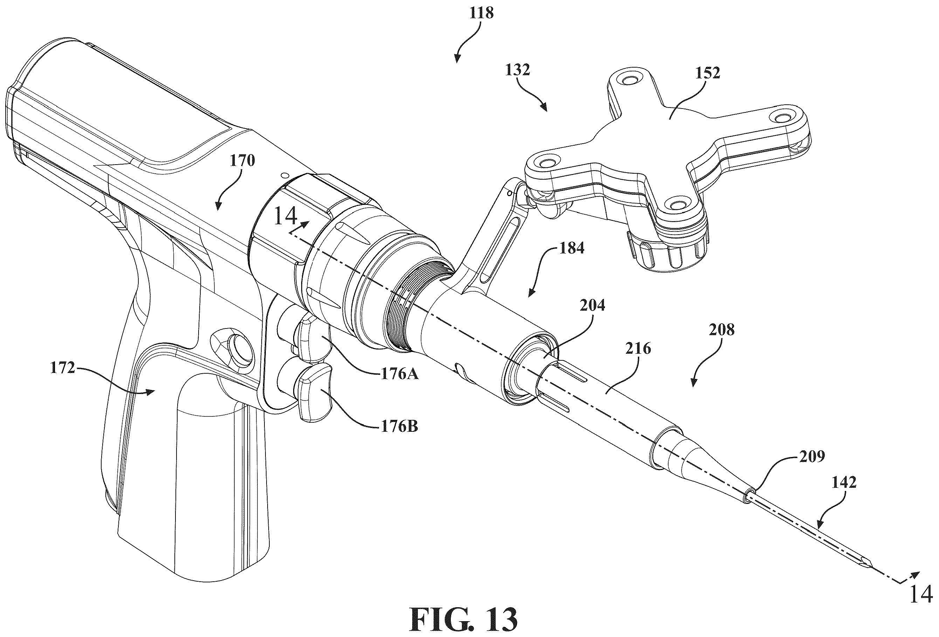

As seen best in Figure 13, when the instrument tracker 132 is engaged with the surgical tool 200, and the surgical tool 200 is inserted into the handpiece 170, the instrument tracker 132 engages both the surgical tool 200 and the handpiece 170. As the proximal portion 206 of the surgical tool 200 into the receiver 178, the retainer 214 of the surgical tool 200 engages the coupling mechanism 180 of the receiver 178, securing the retainer 214 at retention feature 182. With respect to the instrument tracker 132 on the surgical tool 200, as proximal portion 206 of the surgical tool 200 is inserted into the receiver 178, the abutment interface 198 defined by the biasing element 196 of the instrument tracker 132 will engage a front surface of the receiver 178, with the biasing element 196 urging the adapter 184 away from the receiver 178 and causing the adapter 184 to abut the stop 210 of surgical tool 200. Once the adapter 184 abuts the stop 210, the biasing element 196 compresses as the proximal portion 206 of the surgical tool 200 is inserted further into the receiver 178 until the coupling mechanism 180 engages the retainer 214, thereby securing the surgical tool 200 to the surgical handpiece 170. The detent of the coupler 197 on the adapter 184 is seated in the recess 212 defined in the body 204 of the surgical tool 200 to secure the adapter 184 to the surgical tool 200. Prior to attaching the surgical tool 200 to the surgical handpiece 170, the surgical tool 200 and adapter 184 are configured such that the adapter 184 may spin freely about the body 204.

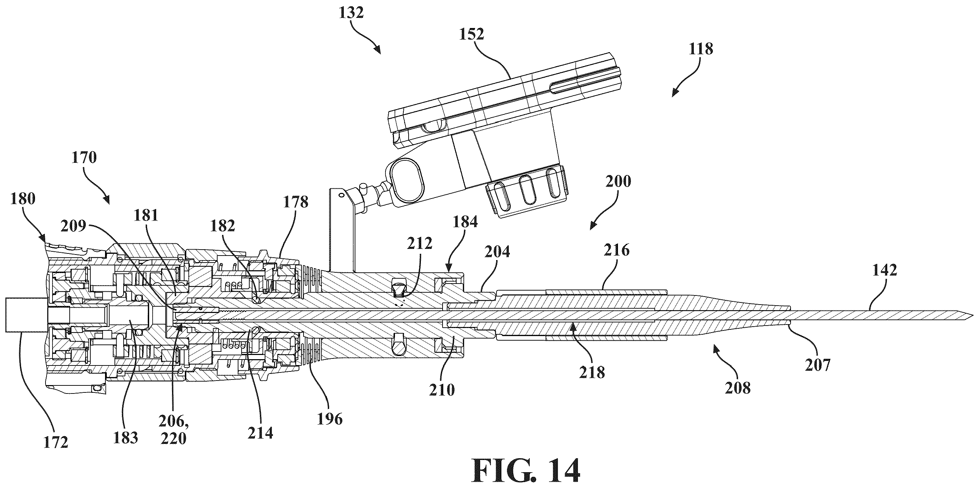

As the proximal portion 206 of the surgical tool 200 is inserted into the receiver 178, the proximal portion 206 engages the coupling mechanism 180 of the receiver 178. As best seen in Figure 14, the proximal end 207 of the proximal portion is inserted into the spindle bore 181 until the proximal end 207 abuts a drive assembly. The drive assembly may include a motor 172 in communication with a driveshaft 183. Driveshaft 183 may be connected with a motor to provide rotational power to the driveshaft 183 and spindle bore 181, which is configured to rotate the surgical tool 200. Examples of the receiver 178 and handpiece 170 of these types are described in U.S. Patent No. 9,192,394, entitled *"Surgical Handpiece with Compact Clutch,"* the disclosure of which is hereby incorporated by reference in its entirety.

The proximal portion 206 of the surgical tool 200 is inserted into the spindle bore 181 of receiver 178 such that the spindle bore 181 accepts and supports the proximal portion 206 of the surgical tool 200, and subsequently, the proximal portion of the fixation device 142. The support provided by the spindle bore 181 and the receiver 178 prevents bending of the fixation device 142 during placement and facilitate accurate navigation of the fixation device 142 to the planned placement.

Those having ordinary skill in the art will appreciate that aspects of the embodiments described and illustrated herein can be interchanged or otherwise combined.

It will be further appreciated that the terms "include," "includes," and "including" have the same meaning as the terms "comprise," "comprises," and "comprising." Moreover, it will be appreciated that terms such as "first," "second," "third," and the like are used herein to differentiate certain structural features and components for the non-limiting, illustrative purposes of clarity and consistency.

Several configurations have been discussed in the foregoing description. However, the configurations discussed herein are not intended to be exhaustive or limit the invention to any particular form. The terminology which has been used is intended to be in the nature of words of description rather than of limitation. Many modifications and variations are possible in light of the above teachings and the invention may be practiced otherwise than as specifically described.

## Claims

1. A surgical system (100) for navigated surgery comprising:
a surgical power tool (118) including a housing including a motor (172) and a receiver (178), the motor (172) disposed within the housing, the motor (172) operatively connected with the receiver (178), the receiver (178) configured to removably receive a tool;
a tool (200) including:
a body (204) with a distal portion (208) with a distal end (209) and a proximal portion (206) with a proximal end (207), the proximal portion (206) of the body (204) configured to be selectively received by the receiver (178) of the surgical power tool (118);
a lumen defined through the body (204) by an inner wall (218) forming a void and extending between the distal end (209) and the proximal end (207) of the body (204); and
a retention assembly (220) configured to selectively retain a fixation device (142); and
a navigation adapter (184, 184A);
a fixation device (142);
wherein the proximal end (207) of the tool (200) is positioned within the receiver (178) of the surgical power tool (118);
wherein the fixation device (142) is retained by the retention assembly (220) in a position such that the proximal end (207) of the fixation device (142) is positioned within the surgical power tool (118).

2. The system of claim 1, wherein the receiver (178) of the surgical power tool (118) is configured to impart rotational movement from the motor (172) to the tool (200), wherein the tool (200) is configured to apply a torque to the fixation device (142) through a drive portion (144).

3. The system of claim 2, wherein the drive portion (144) includes a keyed cross-section and a retention assembly (220), the retention assembly (220) includes a biasing element (196);
wherein the proximal end (207) of the fixation device (142) includes a geometry substantially matching the keyed cross-section of the drive portion (144).

4. The system of claim 2, wherein the drive portion (144) is within the proximal portion (206) of the lumen.

5. The system of claim 4, wherein an inner diameter of the drive portion (144) is sized and shaped to support a length of the proximal portion (206) of the fixation device (142).

6. The system of claim 5, wherein the proximal portion (206) of the tool (200) includes an outer profile that is sized and shaped to be received within and supported by the surgical power tool (118), the outer profile of the proximal portion (206) including a first outer diameter configured to interface with a spindle of the receiver (178) of the surgical power tool (118), and a second outer diameter is greater than the first outer diameter configured to interface with a retainer (214) of the surgical power tool (118);
wherein, as an option, the outer profile of the proximal end (207) of the tool (200) further includes a third outer diameter greater than the second outer diameter and configured to receive the navigation adapter (184, 184A), and a fourth outer diameter larger than the third outer diameter and configured as a positional stop (210) for the navigation adapter (184, 184A).

7. The system of any one of claims 1 to 6, wherein the distal portion (208) of the tool (200) includes a handle (216) section and a nose tube, the handle (216) section having a handle (216) outer diameter and the nose tube having a tapered diameter reducing in size from the handle (216) outer diameter to the distal end (209) of the tool (200).

8. The system of any one of claims 1 to 7, wherein the distal portion (208) of the tool (200) includes a first inner diameter extending through the distal portion (208) of the tool (200) to the distal end (209) of the tool (200) providing limited radial compliance with the fixation device (142) to support the proximal portion (206) of the fixation device (142);
wherein, as an option, the body (204) of the tool (200) includes a second inner diameter extending from the proximal portion (206) to the distal portion (208), and second inner diameter being larger than the first inner diameter.

9. The system of any one of claims 1 to 8 wherein the body (204) of the tool (200) comprises a first body (204) corresponding to the proximal portion (206) and a second body (204) corresponding to the distal portion (208).

10. The system of any one of claims 1 to 9, further comprising a navigation adapter (184, 184A) configured to support a navigation array, the navigation adapter (184, 184A) coupled to the tool (200) and the surgical power tool (118); and/or
wherein the fixation device (142) has a length longer than the tool (200); and/or
wherein the fixation device (142) is a guide wire.

11. A surgical tool (200) configured to drive a fixation device (142) in a patient (112), the tool (200) comprising:
a body (204) with a distal portion (208) and a proximal portion (206);
a lumen defined through the body (204) by an inner wall (218) forming a void between a distal end (209) of the distal portion (208) and a proximal end (207) of the proximal portion (206) of the body (204), the inner wall (218) configured to support a length of the fixation device (142) within the lumen;
a retention assembly (220) configured to selectively retain a fixation device (142);
a sleeve disposed about a portion of the distal end (209) of the body (204); and
wherein the inner wall (218) supports the fixation device (142) at the proximal end (207) and distal end (209) of the tool (200), the lumen configured to receive a proximal portion (144) and a distal portion (148) of the fixation device (142) such that the inner wall (218) supports the proximal portion (144) and the distal portion (148) of the fixation device (142).

12. The surgical tool (200) of claim 11, wherein the distal portion (148) of the fixation device (142) is supported by the tool (200) at least at a distal portion (148) of the fixation device (142).

13. The surgical tool (200) of claim 11 or 12, wherein the inner wall (218) supports at least part of the distal portion (148) of the fixation device (142), such that the distal portion (148) of the fixation device (142) extends past the distal end (209) of the body (204) while a part of the distal portion (148) of the fixation device (142) is within the body (204) of the distal portion (208) of the tool (200) supported by the inner wall (218).

14. The surgical tool (200) of any one of claims 11 to 13 wherein the tool (200) is configured to receive a navigation adapter (184, 184A) selectively positionable about circumference of the body (204) of the tool.

15. The surgical tool (200) of any one of claims 11 to 14 wherein the distal portion (208) of the tool (200) includes an inner diameter extending to the distal end (209) of the tool (200), the inner diameter providing limited radial compliance with the fixation device (142) to support the proximal portion (144) of the fixation device (142); and/or
wherein the body (204) includes a drive portion (144), the drive portion configured to rotate the fixation device (142) with the tool (200).
